(19) Europäisches Patentamt
European Patent Office
Office européen des brevets

(11) Publication number: **0 326 520 A2**

# (12) EUROPEAN PATENT APPLICATION

(21) Application number: 89810062.3

(22) Date of filing: 25.01.89

(51) Int. Cl.[4]: C 07 D 213/30
C 07 D 213/61,
C 07 D 213/64,
C 07 D 213/70,
C 07 D 213/09,
C 07 D 239/26,
C 07 D 333/16,
C 07 D 307/42,
C 07 D 307/46,
C 07 D 263/14, C 07 D 215/14
// C07D215/18, C07D307/38,
A01N43/08, A01N43/10,
A01N43/40, A01N43/42,
A01N43/54, A01N43/76,
C07C83/10

(30) Priority: 29.01.88 US 149794

(43) Date of publication of application:
02.08.89 Bulletin 89/31

(84) Designated Contracting States:
AT BE CH DE ES FR GB GR IT LI LU NL SE

(71) Applicant: SANDOZ AG
Lichtstrasse 35
CH-4002 Basel (CH)

(84) Designated Contracting States:
BE CH ES FR GB GR IT LI LU NL SE

(71) Applicant: SANDOZ-PATENT-GMBH
Humboldtstrasse 3
D-7850 Lörrach (DE)

(84) Designated Contracting States: DE

(71) Applicant: SANDOZ-ERFINDUNGEN
Verwaltungsgesellschaft m.b.H.
Brunner Strasse 59
A-1235 Wien (AT)

(84) Designated Contracting States: AT

(72) Inventor: Baum, John William
922 El Cajon Way
Palo Alto, CA 94303 (US)

Cloudsdale, Ian Stuart
730 Rebecca Drive
Boulder Creek, CA 95006 (US)

Lee, Ming
166 Sweetberry Court
San Jose, CA 95136 (US)

Pomes, James Christopher
38466 Berkeley Common
Fremont, CA 94536 (US)

Claims for the following Contracting States: ES + GR.

## (54) Hydrazides and hydroxamic acid derivatives.

(57) The present invention relates to hydrazides and hydroxamid acid derivatives of formula I and formula II

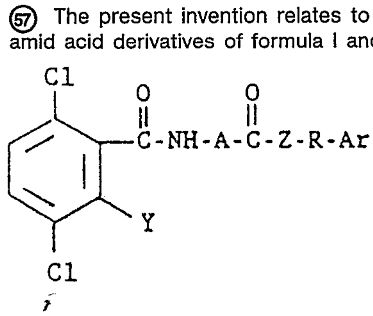

(I)

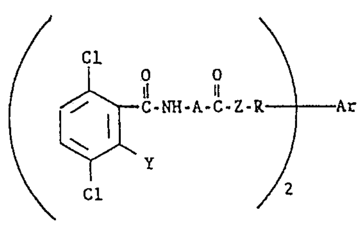

(II)

wherein Y, A, Z, R and Ar are as defined in the specification, their use as herbicides and to agricultural compositions containing the same.

EP 0 326 520 A2

## Description

## HYDRAZIDES AND HYDROXAMIC ACID DERIVATIVES

The present invention relates to hydrazides and hydroxamic acid derivatives of benzoic acid, their use as herbicides and to agricultural compositions containing the same.

The present invention more particularly relates to compounds of formula I or II:

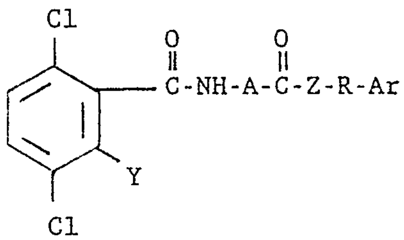

(I)

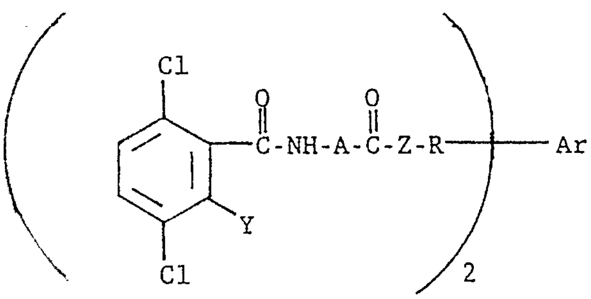

(II)

wherein Y is Cl or $OCH_3$;
A is O-alkylene of 1 to 5 carbon atoms, O-alkenylene of 3 to 6 carbon atoms in which the unsaturation is non-adjacent to the oxygen atom thereof or NH-alkylene of 1 to 5 carbon atoms, the O- and NH- thereof being attached to the NH which is adjacent to A;
Z is oxygen or sulfur;
R is $C_{1-6}$alkyl;
Ar is an optionally substituted heterocyclic ring system selected from

a) a heterocycloalkyl group of 3 to 6 carbon atoms and 1 to 3 hetero atoms chosen from O, S or NH, optionally substituted with $C_{1-6}$alkyl, hydroxyl or halogen;

b) a heterocycloalkenyl group of 3 to 6 carbon atoms and 1 to 3 hetero atoms chosen from O, S or NH, optionally substituted with $C_{1-6}$alkyl, hydroxyl or halogen;

c) a group of formula X or XI

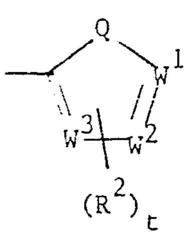

(X)

or

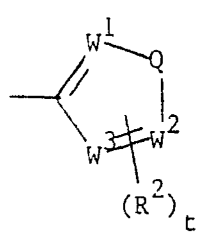

(XI)

wherein Q is O, S or $NR^1$; and each of $W^1$-$W^3$ is independently CH or N;

d) a group of formula XII

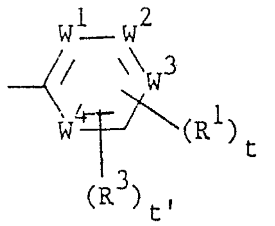

(XII)

wherein each of $W^1$-$W^4$ is independently CH, N or N→O; with the proviso that at least one of $W^1$-$W^4$ is N and not all of $W^1$-$W^3$ are N; or

e) a group of formula XIII

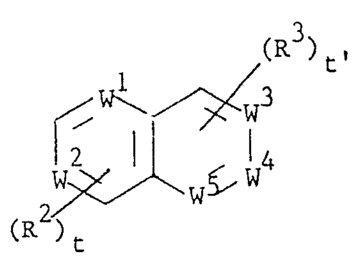

(XIII)

wherein, each of $W^1$-$W^5$ is independently CH or N; with the proviso that at least one of $W^1$ and $W^2$ is N and not all of $W^3$-$W^5$ are N;

$R^1$ is hydrogen; $C_{1-8}$alkyl optionally substituted with 1 to 6 halogen atoms or with a hydroxyl group; or optionally substituted aryl;

each of $R^2$ and $R^3$ is independently selected from the values of $R^1$ or $C_{3-8}$cycloalkyl, acyl, halogen, nitro, cyano, $OR^1$, $NHR^1$, $N(R^1)_2$, $COOR^1$, $OCOR^1$, $S(O)_nR^1$ or optionally substituted phenyl; and

n is 0, 1 or 2;

t is zero or one; and

t' is zero, one or two.

When in the compounds of the formula I or formula II, A is O-alkylene or NH-alkylene, the alkylene may be straight chain or branched. Any such branching, e.g. methyl groups, may occur once or twice on any carbon atom of the linear portion of the alkylene moiety. Preferably, the alkylene portion of the O-alkylene moiety is of 1 to 3 carbons and contains no more than a single methyl branch, or is unbranched.

When A is O-alkenylene, the alkenylene portion may also be straight chain or branched. Preferably, the connecting linear alkenylene portion is of 3 or 4 carbon atoms and contains no more than a single methyl branch or is unbranched, and is more preferably unbranched, e.g. allylene.

Preferred compound groups are those of formula I or formula II is which;

Y is $OCH_3$

A is $C_{1-3}$O-alkylene, more preferably $OCHR^1$ wherein $R^1$ is H or methyl and most preferably $OCH_2$.

Z is oxygen

R is $C_{1-4}$alkyl, straight chain or branched, more preferably $C_{1-2}$alkyl and most preferably $CH_2$.

Ar is optionally substituted thienyl, furyl, 1,3-oxazolinyl, pyridyl, pyridyl-N-oxide or quinolyl. Where such Ar is substituted it is preferably substituted by one or two substituents selected from halogen, $C_{1-4}$alkyl, $C_{1-4}$alkoxy, $C_{1-4}$alkylthio and phenyl.

When in salt form, the alkali metal and ammonium salts are generally preferred, particularly the sodium and potassium salts, or the ammonium salt forms including the secondary and tertiary ammonium salt forms, such as dimethylammonium salt, isopropylammonium salt, diethanolammonium salt, triethanolammonium salt and the 2-hydroxyethyloxyethylammonium salt. Other salt forms which may be prepared include the hydrazinium salt forms which may be derived from unsubstituted or substituted hydrazine, e.g. hydrazine, $NH_2N(CH_2CH_3)_2$ and the like.

A particularly preferred compound of the present invention is furfuryl-[(3,6-dichloro-2-methoxybenzoyl)aminooxylacetate.

The compounds of the formula I and of formula II in free form or in salt form may be prepared by

a) reacting a compound of the formula III:

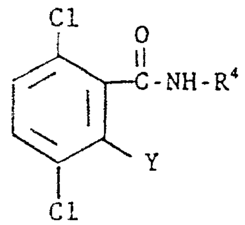

(III)

wherein $R^4$ is OH or $NH_2$,
and Y is as defined above,
with a compound of formula IV or IVa
X-A′-CO-ZR-Ar (IV)
or $(X\text{-}A'\text{-}CO\text{-}Z\text{-}R)_2Ar$ (IVa)
wherein X is halogen, preferably bromo or chloro; A′ is alkylene of 1 to 5 carbons or alkenylene of 3 to 6 carbons in which the unsaturation is non-adjacent to X; and Z, R and Ar are as defined above, and optionally reacting the thus obtained compounds of formula I or formula II with a salt-forming base.

The preparation of the hydroxamic acid derivatives of formula I and formula II by process a) is conveniently carried out at temperatures of from about 25° C to 150° C, preferably 60° C to 120° C, in the presence of a base and in a solvent media. Preferred bases are the alkali metal hydroxides such as sodium hydroxide or potassium hydroxide. Preferred solvents are the lower alkanols such as methanol or ethanol or a mixture of water and a lower alkanol, e.g. water and ethanol.

The preparation of the hydrazides of formula I and formula II by process a) is conveniently carried out at temperatures of from about 20° C to 120° C, preferably 40° C to 90° C, in the presence of a base and in a solvent media. A typically preferred base for such reaction is sodium carbonate. Preferred solvents include the acyclic and cyclic ethers such as tetrahydrofuran.

Compounds of formula I may alternatively be prepared by
b) reacting a compound of formula V:

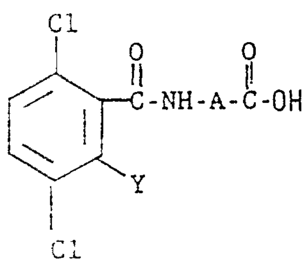

(V)

wherein Y and A are as defined above,
with a compound of formula VI or VIa:
HZ-R-Ar (VI)
or $(HZ\text{-}R)_2Ar$ (VIa)
wherein Z , R and Ar are as defined above, and optionally reacting the thus obtained compounds of formula I or formula II with a salt-forming base.

The preparation of the compounds of formula I and formula II by process b) may conveniently be carried out at room temperature or slightly above or below room temperature in the presence of a condensing agent such as dicyclohexylcarbodiimide and optionally a catalytic amount of base such as dimethylaminopyridine in a suitable solvent which is inert under the reaction conditions, such as ether or dichloromethane.

It will be appreciated that the preparation of compounds of formula II according to process a), i.e. by reaction of compound III with compound IVa, will give optimum yields when employing two equivalents of a compound of formula III. Analogously the preparation of compounds of formula II by reaction with compound V with compound VIa, will give optimum yields when employing two equivalents of a compound of formula V.

The compounds of formula I have the tautomeric form:

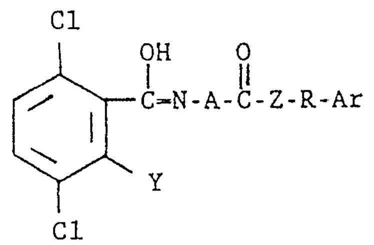

wherein A, Z, Y, R and Ar are as defined. Such enols of the compounds I are of acid character and form, and accordingly the compounds I will also form salts. Analogously, the compounds of formula II may exist in their enol form and form salts.

The compounds of formulae I and II and their salts may be recovered from the reaction mixture by standard procedures.

The salt forms may be obtained from the free acid enol form according to known procedures and vice versa.

The compounds of the formula III may be prepared from the corresponding benzoic acid chloride. Insofar as the production of starting materials used in preparation of the compounds of the formula I and formula II and their salts is not particularly described, these compounds are either known or may be prepared from known materials by conventional methods.

The compounds of the formula I and formula II (including the agriculturally acceptable salts thereof) are useful because they control the growth of plants. By "plants" it is meant germinating seeds, emerging seedlings and established vegetation including underground portions. In particular, the compounds are useful as herbicides as indicated by causing damage to both monocotyledoneous and dicotyledoneous plants in various standard evaluations for determining such effects. The herbicidal effects are exhibited both pre- and post- emergence. Such herbicidal effects indicate that the compounds of formula I and formula II are particularly of interest in combatting weeds (unwanted plants) in a locus in which such weeds are present.

The compounds of formula I and formula II are indicated mainly to be stronger-acting against dicotyledoneous plants than monocotyledoneous plants. Relatively less toxicity towards crops than towards weeds is further indicated. Hence, the compounds are of particular interest as selective herbicides to combat weeds in a crop locus, particularly a locus of a monocotyledoneous crop such as, for example, corn (maize), oats, rice, wheat, sorghum and the like, especially corn.

Additionally, the compounds of formula I and formula II are indicated to have greater activity when applied to the locus pre-emergence than when applied post-emergence. The compounds of the invention are accordingly indicated for use particularly as pre-emergence herbicides against monocotyledoneous and dicotyledoneous weeds, more particularly against dicotyledoneous weeds.

The compounds of the present invention are biologically different from dicamba, in that they are primarily indicated for use as pre-emergence herbicides, whereas dicamba is essentially a post-emergence herbicide. The compounds are also indicated to be more persistent and less mobile in the soil than dicamba, which is advantageous from the efficacy and ecological point of view. Dicamba is 3,6-dichloro-2-methoxybenzoic acid, U.S. Patent 3,013,054.

The present invention therefore also provides a method of combatting weeds in a locus which comprises applying to the locus a herbicidally effective amount of a compound of the invention. When selective action is desired in a crop locus, the amount applied will be sufficient to combat weeds without substantially damaging the crop.

For general herbicidal as well as selective herbicidal use of the compounds of the invention, the particular amounts to be applied will vary depending upon recognized factors such as the compound employed, the plants primarily in the locus, the timing, mode and formulation in application, the various conditions of treatment such as soil and weather and the like. However, in general, satisfactory results in weed control are usually obtained upon application of the compounds of the invention at a rate in the range of 0.1 to 10 kg/hectare, more usually 0.3 to 5 kg/hectare, and preferably 0.5 to 3kg/hectare, the application being repeated as necessary. When used in crops, the application usually will not exceed about 5 kg/hectare, and is usually in the range of 0.1 to 4 kg/hectare, preferably 0.5 to 3 kg/hectare.

For practical use as herbicides, the compounds of formula I and formula II may be and are preferably employed in herbicidal compositions comprising a herbicidally effective amount of the compound and an inert carrier which is agriculturally acceptable in the sense of not, by reason of its presence, poisoning the agricultural environment including the immediate soil of application or any crops present therein or otherwise being unsafe for application. Such compositions of formulations may contain 0.01; to 99% by weight of active ingredient, from 0 to 20% by weight of agriculturally acceptable surfactants and 1 to 99.99% by weight of the inert carrier. Higher ratios of surfactant to active ingredient are sometimes desirable and are achieved by incorporation into the formulation or by tank mixing. Application forms of composition typically contain between 0.01 and 25% by weight of active ingredient, but lower or higher levels of active ingredient can, of course, be present depending on the intended use and the physical properties of the compound. Concentrate

forms of composition intended to be diluted before use generally contain between 2 and 90%, preferably between 10 and 80% by weight of active ingredient.

Useful compositions or formulations of the compounds of the invention include dusts, granules, pellets, suspension concentrates, wettable powders, emulsifiable concentrates and the like. They are obtained by conventional manner, e.g. by mixing the compounds of the invention with the inert carrier. More specifically, liquid compositions are obtained by mixing the ingredients, fine solid compositions by blending and, usually grinding, suspensions by wet milling, and granules and pellets by impregnating or coating (preformed) granular carriers with the active ingredient or by agglomeration techniques.

For example, dusts can be prepared by grinding and blending the active compound with a solid inert carrier such as talc, clay, silica and the like. Granular formulations can be prepared by impregnating the compound, usually dissolved in a suitable solvent, onto and into granulated carriers such as the attapulgites or the vermiculites, usually of a particle size range of from about 0.3 to 1.5 mm. Wettable powders, which can be dispersed in water or oil to any desired concentration of the active compound, can be prepared by incorporating wetting agents into concentrated dust compositions.

Alternatively, the compounds of the invention may be used in micro-encapsulated form.

Agriculturally acceptable additives may be employed in the herbicidal compositions to improve the performance of the active ingredient and to reduce foaming, caking and corrosion.

"Surfactant" as used herein means agriculturally acceptable material which imparts emulsifiability, spreading, wetting, dispersibility or other surface-modifying properties. Examples of surfactants are sodium lignin sulphonate and lauryl sulphate.

"Carrier" as used herein means a liquid or solid material used to dilute a concentrated material to a usable or desirable strength. For dusts or granules it can be e.g. talc, kaolin or diatomaceous earth; for liquid concentrate forms, a hydrocarbon such as xylene or an alcohol such as isopropanol; and for liquid application forms, e.g. water or diesel oil.

The compositions of this invention can also comprise other compounds having biological activity, e.g. compounds having similar or complementary herbicidal activity or compounds having antidotal, fungicidal or insecticidal activity.

The following examples are provided to illustrate the practice of the present invention. Temperature is given in degrees Celsius. RT means room temperature.

## FINAL PRODUCTS

### Example 1

To a solution of [(3,6-dichloro-2-methoxybenzoyl)-aminooxy]acetic acid (3.68 g, 12.5 mmol) in 200 ml of dry ether is added 2-hydroxymethylpyridine (1.21 ml, 12.5 mmol), dicyclohexylcarbodiimide (DCC) (3.22 g, 15.6 mmol) and 50 ml of ether. The mixture is stirred at RT for ca. 36 hr. It is then filtered and the solid is washed with ether. The ether is evaporated and the residue is washed in ethyl acetate. The combined organic layers are washed with water and filtered and the solvent is evaporated. Ether is added to the residue, with warming, and the resulting solution is cooled to give an oil. The residue is purified by column chromatography and crystallization to give 2-pyridylmethyl [(3,6-dichloro-2-methoxybenzoyl)aminooxy]acetate (compound 1 under Table A).

### Example 2

To a solution of 2-hydroxymethyl-6-methylpyridine (1.54 g, 12.5 mmol) in 100 ml of ether is added [(3,6-dichloro-2-methoxybenzoyl)aminooxy]acetic acid (3.68 g, 12.5 mmol) in 100 ml of ether, dimethylaminopyridine (DMAP) (0.15 g, 1.25 mmol), DCC (3.22 g, 15.6 mmol) and 50 ml of ether. The mixture is stirred at RT overnight and is then filtered. The solid is washed with ether, which is then evaporated. The oily residue is dissolved in 50% ethyl acetate/hexane and purified by column chromatography and recrystallization to give 6-methyl-2-pyridylmethyl [(3,6-dichloro-2-methoxybenzoyl)aminooxy]-acetate (compound 2 under Table A).

### Example 3

Following the procedures of Example 1 or 2, each of compounds 3-17 under Table A is prepared from the corresponding carboxylic acid and heterocyclic-substituted alkanol.

### Example 4

Following the procedures of Example 1 or 2, each of compounds 18-29 under Table B is prepared from the corresponding carboxylic acid and heterocyclic-substituted alkanol.

### Example 5

DCC (3.22 g, 15.6 mmol) and 50 ml of ether are added to a mixture of [(3,6-dichloro-2-methoxybenzoyl)aminooxy]acetic acid (3.68 g, 12.5 mmol), 2,6-(dihydroxymethyl)pyridine (0.87 g, 6.25 mmol) and DMAP (0.15 g, 1.25 mmol) in 200 ml of ether, and the mixture is stirred at RT overnight. The reaction mixture is filtered, and the solid is washed with ether, dried and then added to 200 ml of ethyl acetate and heated to reflux for 1 hr. The mixture is cooled, filtered and washed with ethyl acetate. The solvent is evaporated and the residue is purified to give compound 30 under Table C.

In the same manner, [(3,6-dichloro-2-methoxybenzoyl)-aminooxy]acetic acid is reacted with each of 2,5-(dihydroxymethyl)furan and 3,4-(dihydroxymethyl)furan to give, respectively, compound 31 and compound 32 under Table C.

TABLE A

Compounds of formula I in which A = $OCH_2$, Z = 0, R = $(CH_2)m$ and Ar is a group of formula XII wherein t = 1 and t′ = 0

| Cpd | Y | m | $W^1$ | $W^2$ | $W^3$ | $W^4$ | $R^2$ | m.p. (°C) |
|---|---|---|---|---|---|---|---|---|
| 1 | $OCH_3$ | 1 | N | CH | CH | CH | H | 87- 88 |
| 2 | $OCH_3$ | 1 | N | C-$CH_3$ | CH | CH | H | 71- 72 |
| 3 | $OCH_3$ | 1 | CH | N | CH | CH | H | 131-132.5 |
| 4 | $OCH_3$ | 1 | CH | CH | N | CH | H | 137-138 |
| 5 | $OCH_3$ | 1 | CH | N→O | CH | CH | H | 129-131 |
| 6 | $OCH_3$ | 1 | CH | CH | N→O | CH | H | 138-139 |
| 7 | $OCH_3$ | 1 | CH | N | C-Cl | CH | H | 104-106 |
| 8 | $OCH_3$ | 1 | CH | N | C-$CH_3$ | CH | H | 148-149 |
| 9 | $OCH_3$ | 1 | C-$CH_3$ | N | CH | CH | H | 147-148 |
| 10 | $OCH_3$ | 1 | C-$SCH_3$ | N | CH | CH | H | 123.5-125 |
| 11 | $OCH_3$ | 1 | CH | N | CH | CH | Br | 96- 98 |
| 12 | $OCH_3$ | 1 | C-Cl | N | C-Cl | CH | H | 145-150 |
| 13 | $OCH_3$ | 1 | CH | C-$OCH_3$ | N | CH | Cl | 138.5-139 |
| 14 | Cl | 1 | N | CH | CH | CH | H | - |
| 15 | $OCH_3$ | 2 | N | CH | CH | CH | H | oil |
| 16 | $OCH_3$ | 3 | N | CH | CH | CH | H | 96- 98 |
| 17 | $OCH_3$ | 1 | N | CH | CH | N | H | - |
| 33 | $OCH_3$ | 3 | CH | N | CH | CH | H | 89- 90.5 |
| 34 | $OCH_3$ | 3 | CH | CH | N | CH | H | glass |

TABLE B

Compounds of formula I in which A=$OCH_2$, Z=O and R=$(CH_2)m$

| Cpd | Y | m | Ar | m.p. (°C) |
|---|---|---|---|---|
| 18 | $OCH_3$ | 1 | 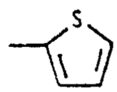 | 84-88 |
| 19 | $OCH_3$ | 1 | 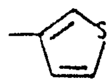 | 87-90 |
| 20 | $OCH_3$ | 1 | 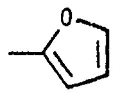 | 86-94 |
| 21 | $OCH_3$ | 1 | 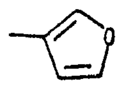 | 96-100 |
| 22 | $OCH_3$ | 1 | 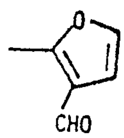 | 93-98 |
| 23 | $OCH_3$ | 1 | 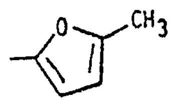 | oil |
| 24 | $OCH_3$ | 1 | 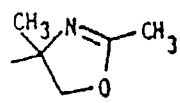 | 103-106 |
| 25 | $OCH_3$ | 1 | 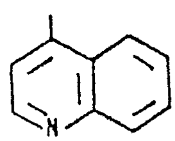 | 188-189 |

TABLE B (cont)

| Cpd | Y | m | Ar | (°C) |
|---|---|---|---|---|
| 26 | $OCH_3$ | 1 | 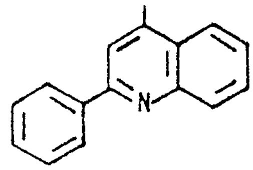 | 73-79 |
| 27 | $OCH_3$ | 1 | 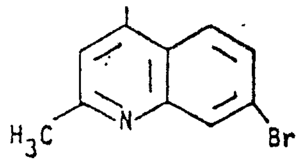 | 175-175.5 |
| 28 | $OCH_3$ | 1 | 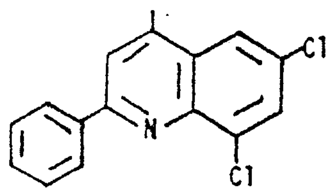 | 176-178.5 |
| 29 | $OCH_3$ | 1 | 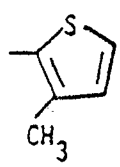 | - |
| 35 | $OCH_3$ | 1 | 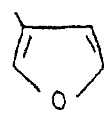 | 78-80 |

TABLE C

Compounds of formula II in which A=$OCH_2$, Z=O, and R=$(CH_2)m$

| Cpd | Y | m | Ar | (°C) |
| --- | --- | --- | --- | --- |
| 30 | $OCH_3$ | 1 | 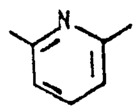 | 141-145 |
| 31 | $OCH_3$ | 1 | 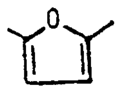 | 148-151 |
| 32 | $OCH_3$ | 1 | 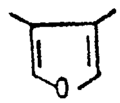 | glass |

TABLE D

Compounds of formula I in which A = $OCH_2$ Z = O, R = $CH(CH_3)$ and Ar is a group of formula XII wherein t = 1 and t′ = 0

| Cpd | Y | $W^1$ | $W^2$ | $W^3$ | $W^4$ | $R^2$ | m.p. (°C) |
| --- | --- | --- | --- | --- | --- | --- | --- |
| 36 | $OCH_3$ | CH | N | CH | CH | H | 130-131.5 |
| 37 | $OCH_3$ | CH | CH | CH | N | H | glass |

TABLE E

Compounds of formula I in which A=$OCH_2$, R=$(CH_2)m$

| Cpd | Y | m | Z | Ar | m.p. (°C) |
| --- | --- | --- | --- | --- | --- |
| 38 | $OCH_3$ | 1 | S | 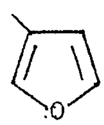 | 89-90 |

INTERMEDIATES

Example A

To 4.0 kg thionyl chloride was added 5.0 kg of 3,6-dichloro-2-methoxybenzoic acid. This was stirred and heated to reflux (ca. 60° C) for 1.5 hours. The reaction mixture was allowed to cool and excess thionyl chloride evaporated in vacuo. The crude product was then distilled under reduced pressure to give 3,6-dichloro-2-methoxybenzoyl chloride as a liquid, b.p. 117° C at 0.6 mm Hg.

Example B

To a solution of 36 g of potassium carbonate in 350 ml of water was added 2.5 litres diethyl ether and 181 g hydroxylamine hydrochloride. The mixture was cooled to 5-10° C and 477 g of 3,6-dichloro-2-methoxybenzoyl chloride was added dropwise at a rate that maintained the reaction temperature below 10° C. After addition

was complete, the reaction mixture was stirred for 1 hour and allowed to stand overnight at ambient temperature. The crude white solid product was obtained by filtration. The crude solid was washed with water, then treated with 1.5 litres of 4.5 M HCl, filtered, washed again with $H_2O$ and dried to give 3,6-dichloro-2-methoxybenzohydroxamic acid as a white solid, m.p. 150°C.

Example C

To a solution of 86.34 g of sodium hydroxide in 1.2 litre of water was added 900 ml of 95% ethanol and 240 g of 3,6-dichloro-2-methoxybenzohydroxamic acid. To aid dissolution, an additional 300 ml of 95% ethanol and 60 ml of $H_2O$ were added. A solution of 153.12 g bromoacetic acid in 120 ml of 95% ethanol was then added to the reaction mixture over 0.5 hours, followed by 60 ml of water. The mixture was then heated to reflux for 3 hours, cooled to ambient temperature, and 150 ml of 20% HCl added. The reaction mixture was then extracted twice with ethyl acetate. The ethyl acetate solutions were combined, washed with brine, and dried ovr $MgSO_4$ overnight, then filtered and solvent evaporated in vacuo. The residue was crystallized from chloroform to yield [(3,6-dichloro-2-methoxybenzoyl)-aminooxy]acetic acid as a white solid (m.p. 145-154°C).

**Claims**

1. A compound selected from formula I or formula II:

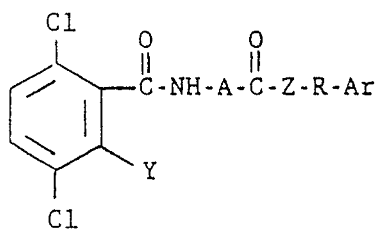

(I)

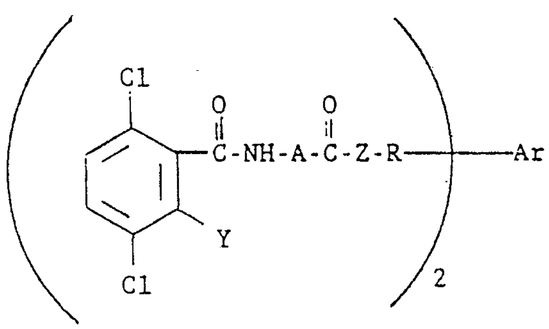

(II)

wherein Y is Cl or $OCH_3$;
A is O-alkylene of 1 to 5 carbon atoms, O-alkenylene of 3 to 6 carbon atoms in which the unsaturation is non-adjacent to the oxygen atom thereof or NH-alkylene of 1 to 5 carbon atoms, the O- and NH- thereof being attached to the NH which is adjacent to A;
Z is oxygen or sulfur;
R is $C_{1-6}$alkyl;
Ar is an optionally substituted heterocyclic ring system selected from

a) a heterocycloalkyl group of 3 to 6 carbon atoms and 1 to 3 hetero atoms chosen from O, S or NH, optionally substituted with $C_{1-6}$alkyl, hydroxyl or halogen;

b) a heterocylcoalkenyl group of 3 to 6 carbon atoms and 1 to 3 hetero atoms chosen from O, S or NH, optionally substituted with $C_{1-6}$alkyl, hydroxyl or halogen;

c) a group of formula X or XI

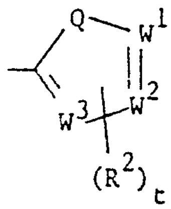 (X) or 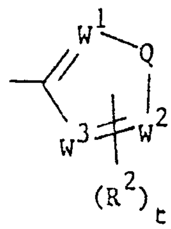 (XI)

wherein Q is O, S or $NR^1$; and each of $W^1$-$W^3$ is independently CH or N;

d) a group of formula XII

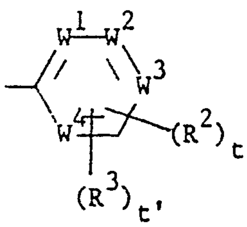 (XII)

wherein each of $W^1$-$W^4$ is independently CH, N, or N→O; with the proviso that at least one of $W^1$-$W^4$ is N and not all of $W^1$-$W^3$ are N; or

e) a group of formula XIII

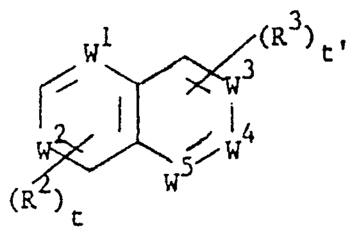 (XIII)

wherein, each of $W^1$-$W^5$ is independently CH or N; with the proviso that at least one of $W^1$ and $W^2$ is N and not all of $W^3$-$W^5$ are N;

$R^1$ is hydrogen; $C_{1-8}$alkyl optionally substituted with 1 to 6 halogen atoms or with a hydroxyl group; or optionally substituted aryl;

each of $R^2$ and $R^3$ is independently selected from the values of $R^1$ or $C_{3-8}$cycloalkyl, acyl, halogen, nitro, cyano, $OR^1$, $NHR^1$, $N(R^1)_2$, $COOR^1$, $OCOR^1$. $S(O)_nR^1$ or optionally substituted phenyl; and

n is 0, 1 or 2;

t is zero or one; and

t' is zero, one or two

in free form or salt form.

2. A compound of formula I according to Claim 1:

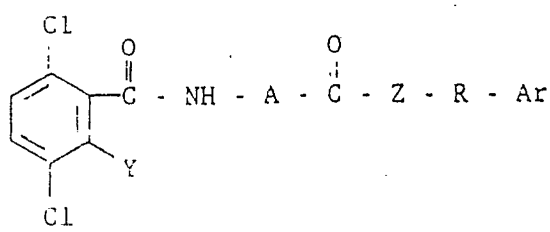

(I)

wherein Y, A, Z, R and Ar are as defined in Claim 1.

3. A compound of formula II according to Claim 1:

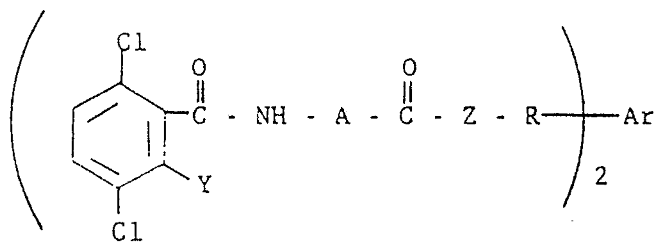

(II)

wherein Y, A, Z, R and Ar are as defined in Claim 1.

4. A compound according to any one of Claims 1 to 3 in which Y is $OCH_3$, A is $C_{1-3}O$-alkylene, Z is oxygen and R is $C_{14}$alkyl.

5. A compound according to Claim 4 in which A is $OCH_2$.

6. A compound according to any one of Claims 2, 4 and 5 in which Ar is a group of formula XII as defined in Claim 1.

7. A compound according to Claim 6 in which R is $CH_2$, $W^1$ is N; each of $W^2$, $W^3$ and $W^4$ is CH and each of $R^2$ and $R^3$ is independently H, methyl, methoxy, methylthio, bromo or chloro.

8. A compound according to Claim 6 in which R is $CH_2$, $W^2$ is N; each of $W^1$, $W^3$ and $W^4$ is CH and each of $R^2$ and $R^3$ is independently H, methyl, methoxy, methylthio, bromo or chloro.

9. A compound according to Claim 6 in which R is $CH_2$, $W^3$ is N; each of $W^1$, $W^2$ and $W^4$ is CH and each of $R^2$ and $R^3$ is,independently H, methyl, methoxy, methylthio, bromo or chloro.

10. A compound according to Claim 6 in which R is $CH(CH_3)$, $W^4$ is N; each of $W^1$, $W^2$ and $W^3$ is CH and each of $R^2$ and $R^3$ is,independently H, methyl, methoxy, methylthio, bromo or chloro.

11. A compound according to any one of Claims 2, 4 and 5 wherein Ar is a group of formula X or XI, as defined in Claim 1.

12. A compound according to Claim 11 in which R is $CH_2$ or $(CH_2)_3$, Q is oxygen or sulphur, $R^2$ is hydrogen, methyl or CHO and each of $W^1$, $W^2$ and $W^3$ is CH.

13. A compound according to any one of claims 2, 4 and 5 wherein R is $CH_2$ and Ar is a group of formula XIII as defined in Claim 1.

14. The compound according to Claim 1 which is furfuryl-[(3,6-dichloro-2-methoxybenzoyl)aminooxy]acetate

15. A process for the preparation of a compound selected from formula I and formula II, as defined in Claim 1, which comprises a) reacting a compound of formula III

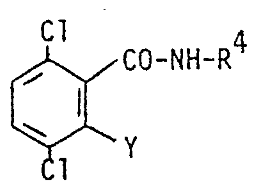

(III)

wherein $R^4$ is OH or $NH_2$
and Y is as stated in Claim 1,
with a compound of formula IV or IVa:
X-A'-CO-Z-R-Ar (IV)

or $(X\text{-}A'\text{-}CO\text{-}Z\text{-}R)_2Ar$ (IVa)
wherein X is halogen,
A' is alkylene of 1 to 5 carbons or alkenylene of 3 to 6 carbon atoms in which the saturation is non-adjacent to X,
and Z, R and Ar are as stated in Claim 1,
or
b) reacting a compound of formula V

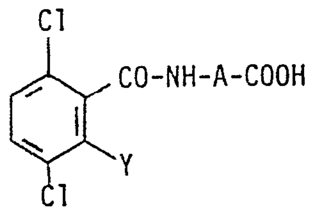

(V)

wherein Y and A are as stated in Claim 1,
with a compound of formula VI or VIa
HZ-R-Ar (VI)
or $(HZ\text{-}R)_2Ar$ (VIa)
wherein Z, R and Ar are as stated in Claim 1,
and optionally reacting the thus obtained compounds of formula I or formula II with a salt-forming base.

16. A method of combatting weeds which comprises applying to the weeds or the locus thereof a herbicidally effective amount of a compound of any one of Claims 1 to 14.

17. A herbicidal composition comprising a compound of any one of Claims 1 to 14.

**Claims for the following contracting States: ES + GR.**

1. A process for preparing compounds selected from formula I or formula II:

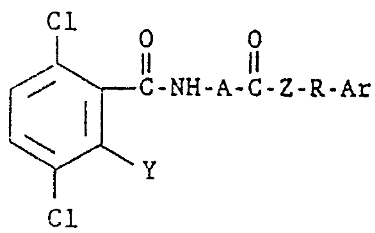

(I)

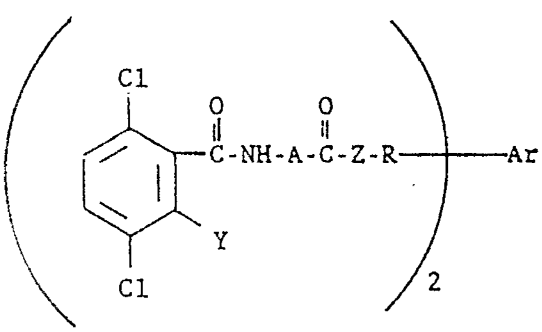

(II)

wherein Y is Cl or $OCH_3$;
A is O-alkylene of 1 to 5 carbon atoms, O-alkenylene of 3 to 6 carbon atoms in which the unsaturation is non-adjacent to the oxygen atom thereof or NH-alkylene of 1 to 5 carbon atoms, the O- and NH- thereof being attached to the NH which is adjacent to A;
Z is oxygen or sulfur;

R is $C_{1-6}$alkyl;

Ar is an optionally substituted heterocyclic ring system selected from

a) a heterocycloalkyl group of 3 to 6 carbon atoms and 1 to 3 hetero atoms chosen from O, S or NH, optionally substituted with $C_{1-6}$alkyl, hydroxyl or halogen;

b) a heterocylcoalkenyl group of 3 to 6 carbon atoms and 1 to 3 hetero atoms chosen from 0, S or NH, optionally substituted with $C_{1-6}$alkyl, hydroxyl or halogen;

c) a group of formula X or XI

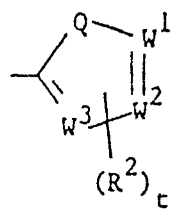

(X)

or

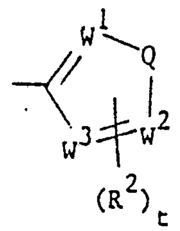

(XI)

wherein Q is O, S or $NR^1$; and each of $W^1$-$W^3$ is independently CH or N;

d) a group of formula XII

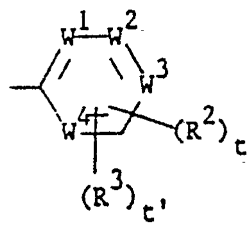

(XII)

wherein each of $W^1$-$W^4$ is independently CH, N or N→O; with the proviso that at least one of $W^1$-$W^4$ is N and not all of $W^1$-$W^3$ are N; or

e) a group of formula XIII

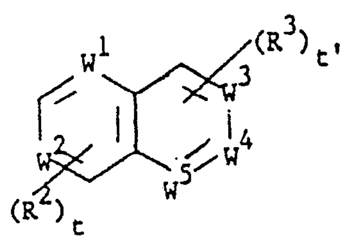

(XIII)

wherein, each of $W^1$-$W^5$ is independently CH or N; with the proviso that at least one of $W^1$ and $W^2$ is N and not all of $W^3$-$W^5$ are N;

$R^1$ is hydrogen; $C_{1-8}$alkyl optionally substitute with 1 to 6 halogen atoms or with a hydroxyl group; or optionally substituted aryl;

each of $R^2$ and $R^3$ is independently selected from the values of $R^1$ or $C_{3-8}$cycloalkyl, acyl, halogen, nitro, cyano, $OR^1$, $NHR^1$, $N(R^1)_2$, $COOR^1$, $OCOR^1$, $S(O)_nR^1$ or optionally substituted phenyl; and

n is 0, 1 or 2;

t is zero or one; and

t′ is zero, one or two

in free form or salt form, which comprises

a) reacting a compound of formula III

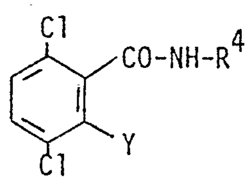

(III)

wherein $R^4$ is OH or $NH_2$
and Y is as stated in this claim,
with a compound of formula IV or IVa:
X-A′-CO-Z-R-Ar (IV)
or $(X\text{-}A'\text{-}CO\text{-}Z\text{-}R)_2Ar$ (IVa)
wherein X is halogen,
A′ is alkylene of 1 to 5 carbons or alkenylene of 3 to 6 carbon atoms in which the saturation is non-adjacent to X,
and Z, R and Ar are as stated in this claim,
or
b) reacting a compound of formula V

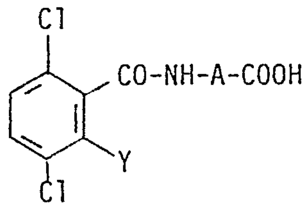

(V)

wherein Y and A are as stated in this claim,
with a compound of formula VI or VIa
HZ-R-Ar (VI)
or $(HZ\text{-}R)_2Ar$ (VIa)
wherein Z, R and Ar are as stated in this claim, and optionally reacting the thus obtained compounds of formula I or formula II with a salt-forming base.

2. A process for preparing a compound of formula I according to Claim 1.

3. A process for preparing a compound of formula II according to Claim 1.

4. A process for preparing a compound of formula I or II according to any one of Claims 1 to 3 in which Y is $OCH_3$, A is $C_{1-3}O$-alkylene, Z is oxygen and R is $C_{1-4}$alkyl.

5. A process according to Claim 4 in which A is $OCH_2$.

6. A process according to any one of Claims 2, 4 and 5 in which Ar is a group of formula XII, as defined in Claim 1.

7. A process according to Claim 6 in which R is $CH_2$, $W^1$ is N; each of $W^2$, $W^3$ and $W^4$ is CH and each of $R^2$ and $R^3$ is independently H, methyl, methoxy, methylthio, bromo or chloro.

8. A process according to Claim 6 in which R is $CH_2$, $W^2$ is N; each of $W^1$, $W^3$ and $W^4$ is CH and each of $R^2$ and $R^3$ is independently H, methyl, methoxy, methylthio, bromo or chloro.

9. A process according to Claim 6 in which R is $CH_2$, $W^3$ is N; each of $W^1$, $W^2$ and $W^4$ is CH and each of $R^2$ and $R^3$ is,independently H, methyl, methoxy, methylthio, bromo or chloro.

10. A process according to Claim 6 in which R is $CH(CH_3)$, $W^4$ is N; each of $W^1$, $W^2$ and $W^3$ is CH and each of $R^2$ is,independently H, methyl, methoxy, methylthio, bromo or chloro.

11. A process according to any one of Claims 2, 4 and 5 wherein Ar is a group of formula X or XI, as defined in Claim 1.

12. A process according to Claim 11 in which R is $CH_2$ or $(CH_2)_3$, Q is oxygen or sulphur, $R^2$ is hydrogen, methyl or CHO and each of $W^1$, $W^2$ and $W^3$ is CH.

13. A process according to any one of claims 2, 4 and 5 wherein R is $CH_2$ and Ar is a group of formula XIII as defined in Claim 1.

14. The process according to Claim 1 which is furfuryl-[(3,6-dichloro-2-methoxybenzoyl)aminooxyl]acetate.

15. A herbicidal composition comprising a compound selected from formula I or formula II as defined in any one of Claims 1 to 14.

16. A method of combatting weeds which comprises applying to the weeds or the locus thereof a herbicidally effective amount of a compound selected from formula I or formula II as defined in any one of Claims 1 to 14.